# EUROPEAN PATENT APPLICATION

(11) **EP 4 498 333 A1**
(43) Date of publication of application: **29.01.2025**
(21) Application number: 23187320.9
(22) Date of filing: 24.07.2023
(51) Int. Cl.: G06V 10/82, G06V 20/69

(54) **IMAGE PROCESSING SYSTEM, MICROSCOPE, TRAINED MACHINE LEARNING ALGORITHM, AND CORRESPONDING METHODS TO PREDICT NUCLEAR LABELING**

(71) Applicant: Leica Microsystems CMS GmbH, 35578 Wetzlar (DE)
(72) Inventor: KAPPEL, Constantin, Wetzlar (DE); SERRA LLETI, José Miguel, Wetzlar (DE); SCHWEIKHARD, Volker, Wetzlar (DE); LAI, Hoyin, Wetzlar (DE)
(74) Representative: Maiwald GmbH

(57) **Abstract**

An image processing system is configured to receive at least one reference image, wherein each reference image is a microscopy image capturing cells of a biological sample, wherein the at least one reference image includes at least one reference labeling directed to a reference cellular compartment of the captured cells. The image processing system is configured to employ a trained deep neural network for processing the at least one reference image to generate a target image, wherein the target image includes a target labeling directed to a target cellular compartment of the captured cells, wherein the at least one reference labeling comprises a fluorescence labeling, wherein the reference cellular compartment is a distributed structure within cells, and wherein the target cellular compartment is the cell nucleus.

## Description

### Technical field

This disclosure relates to methods and systems for processing microscopy images. This disclosure particularly relates to methods and systems for employing machine learning to predict nuclear labeling.

### Background

In fluorescence microscopy, the spatial or spatiotemporal distribution of a specific biomolecule is made visible by fluorescent markers, which are visible against a dark background. Fluorescent markers are dyes with specific binding sites or dyes coupled to antibodies or fluorescent proteins. Fluorescence markers are often chosen so that their distributions specifically marks a particular cellular compartment such as a cell organelle e.g. the cell nucleus. Location or morphology of a cell organelle can be diagnostic of cell state or cell health. Specifically, identifying cell nuclei is usually a first step when analyzing cells in microscopy images. Identifying the cell nucleus is important for fields such as histology and pathology, which rely on identification of specific cell types to diagnose disease or identify abnormalities. The traditional approach for identifying the cell nucleus relies on applying a DNA stain, or a fluorescent transgene expression close to the nucleus. Often, DAPI or Hoechst staining are used for marking nuclei. However, nuclear staining is prone to bleaching, is toxic, and risks altering cell behavior and cell division in particular.

In recent years, deep learning approaches have been applied for processing microscopy images of diverse modalities. Specifically, convolutional neural networks such as U-net (Ronneberger et al. "U-net: Convolutional networks for biomedical image segmentation", Medical Image Computing and Computer-Assisted Intervention (MICCAI), Springer, LNCS, Vol.9351: 234--241) are employed for various tasks such as protein detection and image segmentation of single cells. Deep learning approaches have also been proposed for predicting fluorescent labels from transmitted light images of unlabeled fixed or live biological samples (e.g. Christiansen et al. "In Silico Labeling: Predicting Fluorescent Labels in Unlabeled Images", Cell 173, 792-803). Other approaches apply convolutional neural network architectures to segment nuclei from brightfield microscopy images (e.g. Fishman et al. "Practical segmentation of nuclei in brightfield cell images with neural networks trained on fluorescently labelled samples", Journal of Microscopy 284.1, 2021, 12-24), employ machine learning architectures that can perform classification, segmentation, and prediction of orthogonal imaging modalities on a variety of hyperspectral imaging techniques (e.g. Manifold et al. "A versatile deep learning architecture for classification and label-free prediction of hyperspectral images", Nature machine intelligence 3.4, 2021, 306-315), train a neural network to predict images with significantly increased contrast of a target structure (e.g. Kölln et al. "Label2label: training a neural network to selectively restore cellular structures in fluorescence microscopy" Journal of Cell Science 135.3, 2022), or apply a deep learning based algorithm for 2D cell segmentation (Al-Kofahi et al. "A deep learning-based algorithm for 2-D cell segmentation in microscopy images", BMC bioinformatics 19.1 (2018), 1-11).

### Summary

The inventors have realized that applying methods of machine learning offers a solution for improving workflow in fluorescence microscopy. In this disclosure, an approach of predicting cell nuclear labeling from fluorescent labelings directed to other cell compartments is proposed.

An image processing system comprising one or more processors and one or more storage devices is disclosed. The image processing system is configured to receive at least one reference image, wherein each reference image is a microscopy image capturing cells of a biological sample, wherein the at least one reference image includes at least one reference labeling directed to a reference cellular compartment of the captured cells. The image processing system is further configured to employ a trained deep neural network for processing the at least one reference image to generate a target image, wherein the target image includes a target labeling directed to a target cellular compartment of the captured cells, wherein the at least one reference labeling comprises a fluorescence labeling, wherein the reference cellular compartment is a distributed structure within cells, and wherein the target cellular compartment is the cell nucleus.

The disclosed image processing system hence achieves dye conversion so that fluorescent markers for structures that are distributed over the cell are used to infer the cell nucleus. As a result, nuclear staining is obviated, which frees spectral bandwidth and reduces risk to the examined cells.

According to embodiments, the image processing system may further be configured to process the target image to obtain centroids and shapes of cell nuclei of the captured cells. The at least one reference labeling may be based on cytoskeleton markers or cytosolic markers. The at least one reference labeling may comprise a first fluorescence labeling and a second fluorescence labeling different from the first fluorescence labeling. In this manner, the image processing system may infer the cell nucleus at high precision.

According to embodiments, the at least one reference image may comprise a plurality of reference images, wherein the plurality of reference images may represent a series of optical slices parallel to an optical path of a microscope. Processing the at least one reference image may comprise generating a plurality of target images and generating a three-dimensional reconstruction of the target cellular compartment from the plurality of target images.

The image processing system may further comprising an image analysis system configured to process the target image based on the target labeling, wherein the processing may comprise at least one of performing a semantic image segmentation to obtain class labels for each pixel in the target image, applying a cell segmentation algorithm on the target image, applying distance transforms and thresholding on the target image, obtaining a count of the captured cells, or identifying a cell type of the captured cells. The results of the processing hence allow reducing burden to a user of the microscope by directly providing highly relevant data.

According to further embodiments, the image processing system may be configured to process the target image to provide commands to a microscope to least one of adapt a control flow of the microscope, change imaging modalities used by the microscope, or change a center field of view of the microscope based on the identified cells. The results of the processing can hence be employed as direct feedback to the microscope to alleviate user burden when studying a large number of samples.

The present disclosure further relates to a microscope including the image processing system described above. The microscope is configured to provide the at least one reference image to the image processing system and to display the target image.

The present disclosure also relates to a computer-implemented method for automated image management for a microscope. The method comprises using a microscope to obtain at least one reference image, sending the at least one reference image to a server hosting the image processing system described above, receiving the target image sent from the server at the microscope, and controlling settings of microscope based on processing of the target image.

The present disclosure also relates to a computer-implemented method of training a deep neural network. The method comprises receiving training tuples each comprising at least one reference image and a target image, wherein each reference image is a microscopy image capturing cells of a biological sample, wherein the at least one reference image contains at least one reference labeling directed to a reference cellular compartment of the captured cells and the target image contains a target labeling directed to a target cellular compartment of the captured cells, wherein the at least one reference labeling comprises a fluorescence labeling, wherein the reference cellular compartment is a distributed structure within cells, and wherein the target cellular compartment is the cell nucleus. The method further comprises training the deep neural network on the training tuples to predict the target image from the at least one reference image.

In aspects, the deep neural network may be based on a fully convolutional image-to-image neural network, or may be based on a visual transformer, or may be based on a diffusion model, or may be based on an adversarial network. The training tuples may further comprise bright field microscopy images and phase contrast microscopy images.

The method may further comprise pre-training the deep neural network on pre-training tuples for predicting another target labeling from another reference labeling, the another target labeling and the another reference labeling being different from the target labeling and the reference labeling.

In embodiments in which the deep neural network is based on a variational autoencoder, the method may further comprise pre-training the deep neural network on pre-training tuples in a semi-supervised manner.

The present disclosure also relates to a trained machine learning algorithm trained by receiving training tuples each comprising at least one reference image and a target image, and adjusting the machine learning algorithm based on the training tuples to obtain the trained machine learning algorithm, wherein each reference image is a microscopy image capturing cells of a biological sample, wherein the at least one reference image contains at least one reference labeling directed to a reference cellular compartment of the captured cells and the target image contains a target labeling directed to a target cellular compartment of the captured cells, wherein the at least one reference labeling comprises a fluorescence labeling, wherein the reference cellular compartment is a distributed structure within cells, and wherein the target cellular compartment is the cell nucleus.

### Short Description of the Figures

Embodiments of the present invention are shown in the enclosed drawings in which:
**Figure 1** illustrates use of an image processing system according to an embodiment for predicting nuclear labeling;
**Figure 2** illustrates automated image management for a microscope based on nuclear labeling predicted by an image processing system according to an embodiment;
**Figure 3** illustrates training of an image processing system for predicting nuclear labeling;
**Figure 4** illustrates a flow chart of a method of operating a microscope based on predicted nuclear labeling according to embodiments;
**Figure 5** illustrates a flow chart of a method of training an image processing system for predicting nuclear labeling according to embodiments;
**Figure 6** illustrates a system configured according to embodiments; and
**Figure 7** reproduces microcopy images and output images containing nuclear labeling predicted according to an embodiment.

### Detailed Description

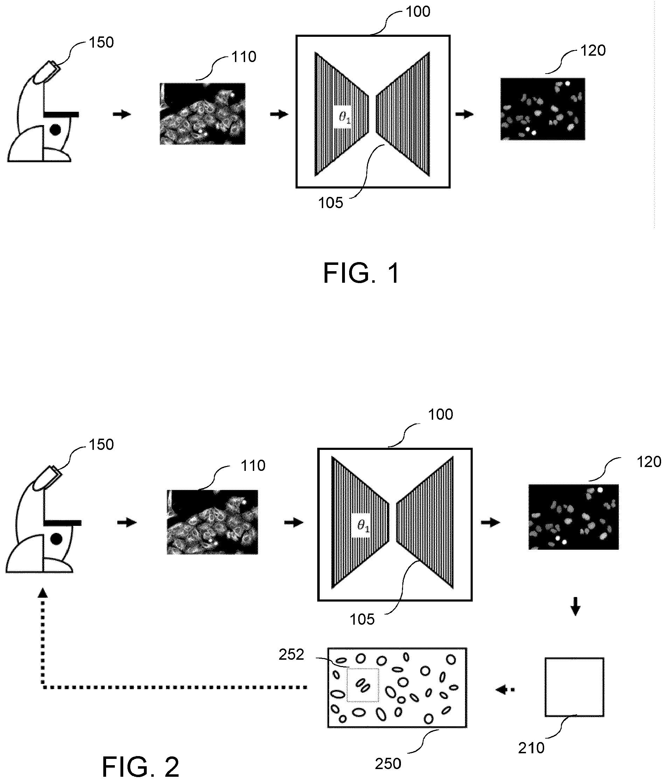

Figure 1 illustrates an image processing system 100 according to embodiments of the invention. The image processing system comprises one or more processors and one or more storage devices. The storage devices store a trained deep neural network 105. The image processing system 100 is configured to receive a reference image 110. The reference image 110 is a microscopy image depicting cells of a biological sample. The reference image 110 can be obtained live from microscope 150 or can be retrieved from storage. The reference image 110 includes a reference labeling directed to a reference cellular compartment of the depicted cell.

The reference labeling may be a fluorescence labeling. By targeting specific proteins that are located next to specific cellular compartments or within these cellular compartments, the reference labeling is directed to the particular compartment and makes the compartment visible in the captured fluorescence light. A cellular compartment comprises all of the closed parts within the cytosol of an Eukaryotic cell, such as cell organelles, mitochondria, chloroplasts, peroxisomes, lysosomes, the endoplasmic reticulum, the cell nucleus, the Golgi apparatus, vesicles, and microtubuli. In embodiments, the reference image 110 may contain a plurality of distinct fluorescence labelings, each directed to a different cellular compartment.

Processors of the image processing system 100 are configured to execute the trained neural network 105 stored in memory to generate a target image 120. Executing the deep neural network 105, based on the parameters *θ*₁ acquired during training, generates target image 120, which contains a target labeling of the target cellular compartment.

The target image includes a target labeling of a target cellular compartment depicted in the biological sample. The target cellular compartment is not among the labeled cellular compartments of the reference images. However, the reference labelings included in the reference image can be chosen such that they allow reliably inferring the target labeling so as to infer position and shape of the target cellular compartment. The image processing system 100 is hence configured to predict from a reference labeling, i.e. a first dye, a target labeling, i.e. a second dye for the cell nucleus, so as to convert the first dye to the second dye.

Identifying cell nuclei is of particular importance in cell biology. The visualization of nuclei allows identifying a cell type. Furthermore, marking of the nuclei in a straightforward manner allows counting the number of cells so that cell growth can be estimated, e.g. to determine effectiveness of a drug treatment. Further, marking of the nucleus allows identifying subcellular localization of proteins surrounding the nucleus to understand the function and activity of these molecules. However, many cell lines available to researchers have been engineered with endogenous markers but not with nuclei markers. Applying additional nuclei dyes however affects behavior of DNA and hence cell division and survival of the cell lines. The disclosed invention allows relying on endogenous markers only while still locating the cell nucleus. Further, in certain research areas such as nuclei damage studies, it is in principle not possible to apply a nuclear dye.

To predict the location and shape of the nucleus, the reference fluorescence labeling must be sufficiently distributed along the cell body so that it conveys information that points to the location and shape of the nucleus. For example, fluorescent labeling for microtubuli and/or actin may be employed. Alternatively, any other cytoskeleton or cytosol labeling may be employed. For example, intermediate filaments may be targeted. The reference image 110 may hence comprise labeling of one or more specific cytoskeleton marker antibodies. In embodiments, the one or more fluorescence labelings included in the one or more reference images may be based on antibody labelings.

In some embodiments, instead of using a single reference image 110, a plurality of reference images are provided to image processing system 100. The plurality of reference images may represent a series of optical slices parallel to an optical path of a microscope. Such plural reference images may correspond to a sequence of microscopy images, each corresponding to a particular focal plane within a thick sample. In these embodiments, a three-dimensional reconstruction of the sample can be achieved so that images perpendicular to the axial dimension are obtained. Employing the generated target images, a three-dimensional reconstruction of the target cell compartment e.g. the cell nucleus can be obtained. In other embodiments, the series of optical slices may also contain a time series.

Figure 2 illustrates an image analysis pipeline for controlling a microscope 150 based on the image processing system 100. Microscope 150 is capturing a microscopy image 110, which is fed to the image processing system 100. The microscope 150 may be an epifluorescence microscope. The microscope may also be based on more advanced designs such as confocal microscope and total internal reflection fluorescence microscope.

In embodiments, image processing system 100 is comprised in the microscope 150. In other embodiments, the image processing system 100 is located at a remote server connected to the microscope 150 via a data network. Microscopy image 110 contains a reference labeling such as a dye directed to a first cellular compartment. The image processing system 100 provides target image 120 including a predicted dye such as nuclear dye. The disclosed microscope 150 is hence enabled to localize nuclei without the need of employing nuclear labeling or nuclear markers.

The microscope 150 may comprise a monitor configured to display the target image 120. The target image 120 may further be provided to an image analysis system 210. The image analysis system 210 is configured to generate an output image 250 including predictions 252 e.g. a region of specific cells recognized in the target image 120. While Figure 2 illustrates image analysis system 210 as being separate from image processing system 100, it is to be understood, that illustrates image analysis system 210 may form part of image processing system 100.

The image analysis system 210 may calculate centroids and shapes of cell nuclei of the captured cells. These data may allow obtaining statistics on a cell nuclei contained in the biological sample. A count of the captured cells can be obtained from the target image 120. Further, labeling of the cell nuclei generated by the deep neural network 105 allows identifying a cell type the captured cells. The image analysis system 210 may accordingly include a machine learning system configured to identify the cell type of the cells depicted in the sample, based on applying a classification on labeled nuclei. The classification may be based on a stored classification for the shapes of the cell nuclei. As is well known, different cell types can have very different nuclear shapes and these shapes have impacts on cellular function. Many pathologies are linked with alterations of the nuclear shape. For example, many leukocytes of the immune system have multi-lobed nuclei, while smooth muscle cells have spindle-shaped nuclei. The nuclear shape has clear effect on the transcriptional activity of the cell. Granulocytes have varying nucleus shapes and have been commonly recognized and distinguished histologically by their nuclear shapes. The shape of the cell nuclei may be measured by geometric measures of the marked cell nuclei.

Further, the image analysis system 210 may be a machine learning system configured for applying a cell segmentation algorithm on the target labeling, such as the target labeling of the cell nucleus. The image analysis system 210 may hence be configured to process the target image 120 to perform a semantic image segmentation. As a result, image pixels corresponding to the cell nucleus may be identified by a first class label and image pixels not belonging to the cell nucleus may be identified by another class label. Semantic image segmentation of cells may also include segmentation of the entire cell body including the cytoplasm. The machine learning model 210 may also be configured to process the target image 120 to perform distance transforms on target image. Based on the labeling of the target cell compartment, a distance of all background points to the nearest object point of the target cell compartment e.g. the cell nucleus can be determined. Processing the target image may also include applying thresholding. The monitor of the microscope may hence also display results of the processing of the target image 120. For example, a count of the captured cells or cells of specific types may be highlighted.

Based on the predictions 252, the microscope 150 may adapt its control flow, change imaging modalities and/or shift a center field of view. For example, a control flow of the microscope 150 may be adapted to analyze another sample if the current sample does not contain a specific cell type. Furthermore, a field of view of the microscope 150 might be adjusted based on the results that the current field of view does not contain a specific cell type. In a similar manner, imaging modalities used by the microscope 150 may be adjusted. Hence, methods of feedback microscopy may be applied to alleviate the tedious task of manually locating specimen by fully integrating the task of identifying and locating a specimen of interest in the image acquisition workload. The present disclosure can also be applied for correlative microscopy, which combines and utilizes information across dimensions, modes, and scales.

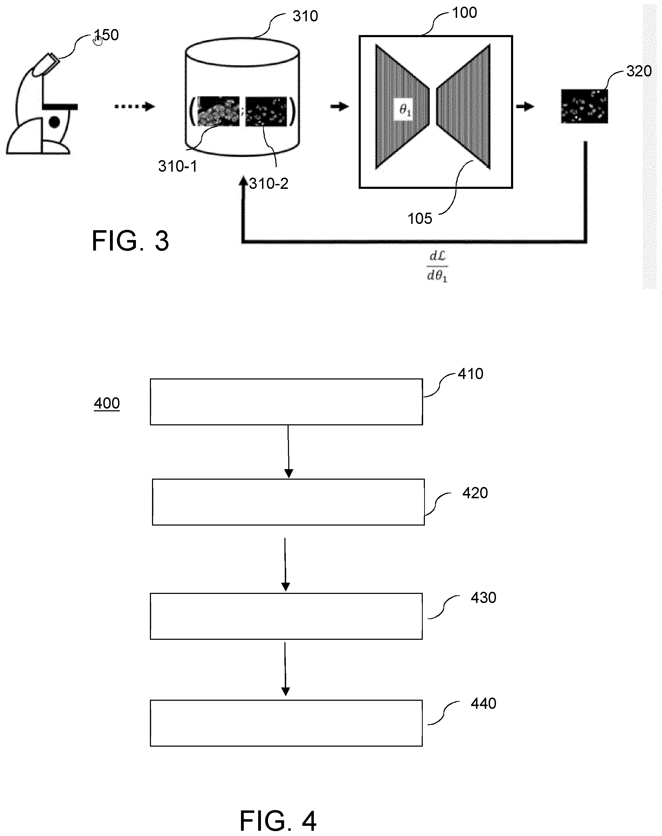

Figure 3 illustrates training of the deep neural network 105 of the image processing system 100. Microscope 150 generates images pairs 310. The image pairs 310 include an image 310-1 in which a source cell compartment, e.g. cell organelle is labeled, and a matching image 310-2 in which the cell nucleus is indicated by a mask or a labeling. In embodiments, both images 310-1 and 310-2 are fluorescence microscopy images. Accordingly, image 310-1 contains a fluorescence labeling directed to reference cell compartments and image 310-2 captures the same sample but contains a labeling directed to the cell nucleus. In alternative embodiments, image 310-2 contains additional labelings or masks. In embodiments, the image 310-2 is a non-light microscopy-based image including a label or mask that allows predicting the cell nucleus.

In embodiments, the deep neural network 105 is a convolutional neural network consisting of an image-to-image pipeline. Training the deep neural network 105 is based on adjusting parameters *θ*₁ of the deep neural network 105. Based on comparing the produced image 320 with the image 310-2, the deep neural network 105 is becoming better at producing output images 320, which correspond to the image 310-2. When correctly trained, the deep neural network 105 is capable of performing inference of the cell nucleus for image scenes not seen during training. When training the deep neural network 105, a loss function is calculated, which can be a mean absolute error, a mean squared error or a binary cross entropy error. The deep neural network 105 can be trained directly for the intended task, or can be pre-trained for a similar task, followed by training for the intended task.

In embodiments, the deep neural network 105 is based on U-net. The U-net architecture consists of a contracting path according to a typical architecture of a convolutional network, and an expansive path, where feature and spatial information are combined through a sequence of up-convolutions and concatenations with high-resolution features from the contracting path. In other embodiments, the deep neural network is a visual transformer (VT). A VT model is based on the architecture of a transformer originally designed for text-based tasks. The VT model may first apply convolution layers to extract low-level features from the input image. A tokenizer may then applied to the feature maps to group the pixels into visual tokens. These tokens are provided to a transformer network to model relationships between the tokens. The transformed patches can be used for image-to-image translation. In still other embodiments, the deep neural network 105 implements an image-to-image diffusion model. Image-to-image diffusion models are conditional diffusion models of the form *p* (***y***|***x***), where both ***x*** and ***y*** are images. Given a training output image ***y***, a noisy version ***ỹ*** is generated. A neural network *f_{θ}* is trained to de-noise ***ỹ***, given ***x*** and a noise level indicator. In yet other embodiments, the deep neural network 105 implements an image-to-image generative adversarial network (GAN). A GAN comprises a generator network and discriminator networks that are trained simultaneously to maximize a joint loss. The objective of the generator network is to generate realistic images in the translated domain that cannot be distinguished from images in the original domain. In yet other embodiments, the deep neural network 105 implements a variational autoencoder (VAE). A VAE is a probabilistic generative model comprising a first neural network, the encoder, which maps the input variable to a latent space that corresponds to the parameters of a variational distribution. A second neural network, the decoder, maps from the latent space to the input space, in order to produce or generate data points. VAEs are particularly suited for semi-supervised learning.

In some embodiments, the deep neural network 105 is pre-trained on a similar task of predicting another target cell compartment from another source cell compartment. In embodiments such as embodiments relying on VAE, the deep neural network can be pre-trained in a semi-supervised manner, leveraging a large corpus of microscopy images, in which only a subset of the corpus contains supervision data i.e. labeling of the target cell compartments e.g. the cell nucleus. The deep neural network 105 can comprise a nonlinear output layer such as a sigmoid layer.

Figure 4 illustrates a flow chart of a method of applying deep neural network for controlling operation of a microscope.

In step 410, at least one reference image of a biological sample is captured by a microscope. The method further comprises sending 420 the at least one reference image via an interface of the microscope to a remote server which comprises the image processing system 100 described above. In these embodiments, the image processing system 100 corresponds to the server and the trained deep neural network 105 forms part of a processing software running on the server. In step 430, the remote server feeds the at least one reference image, in real-time, to the trained deep neural network 105 to generate a target image. The remote server sends the target image back to the microscope.

In step 440, a controller of the microscope evaluates the received target image and adjusts settings of the microscope e.g. regarding imaging modalities, field of view. In embodiments, based on evaluating the received target image, the controller of the microscope provides commands to the microscope to provide the next sample to a sample holder. For processing the target image, the microscope may employ the image analysis system 210 described above.

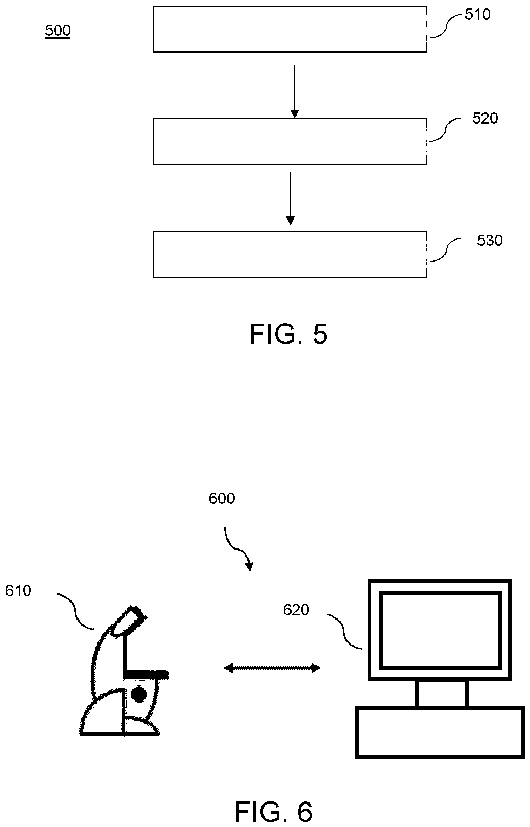

Figure 5 illustrates a flow chart of a computer implemented method 500 of training a deep neural network, such as deep neural network 105, for predicting nuclear labeling. The method comprises receiving 520 training tuples each comprising a reference image and a target image. The reference image and the target image each are microscopy image capturing cells of a biological sample. The reference images contain a reference labeling directed to a reference cellular compartment of the cells. The target image contains a target labeling directed to a target cellular compartment such as the cell nucleus. The reference labeling and the target labeling may be based on fluorescence labeling.

The method further comprises training 530, during which the parameters *θ*₁ of the deep neural network are adjusted based on a difference observed between an output image and a reference image. The difference may be measured by a loss function. The step of training 530 comprises iterating over all of the training samples, e.g. in batches.

In embodiments, the method 500 comprises step 510 of pre-training the deep neural network on a pre-training task for predicting a first target cellular compartment from a first reference cellular compartment which are different from the target cellular compartment and reference cellular compartment trained on during training 530.

In embodiments, the step 510 of pre-training may be based on semi-supervised training. In embodiments, the deep neural network 105 is trained on a large data set of unlabeled microscopy images which may include bright field microscopy images, phase contrast microscopy images or other microscopy modalities without fluorescence labels. The deep neural network is trained to recognize patterns and structures in the images that are useful to infer location and shape of the target cell compartment.

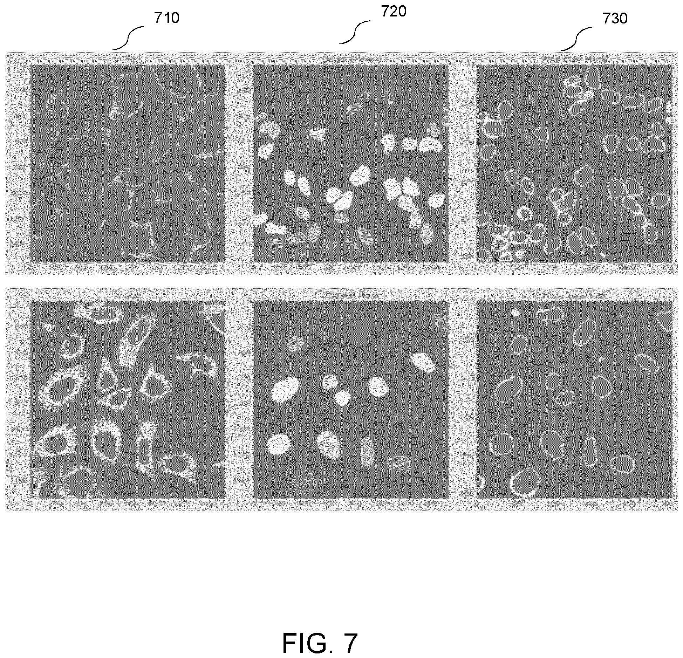

Figure 7 illustrates results obtained by employing an image processing systems 100 according to embodiments and trained in accordance with method 500. Column 710 illustrates exemplary reference images containing a labeling of a reference cell organelle. Column 720 reproduces corresponding training target images containing a labeling of cell nuclei. Column 730 illustrates the generated target images obtained by processing the images of column 710 with the trained deep neural network 105.

When comparing columns 720 and 730, the quality of the disclosed approach can hence be assessed. As shown in the results of Figure 7, the proposed approach allows reliably inferring shape and location of the cell nuclei from a labeling directed to another cell compartment. As a result, the disclosed approach allows saving spectral bandwidth to allow larger number of other labeling/markers to be detected and obvious the need for toxic nuclear staining.

Embodiments may be based on using a machine-learning model or machine-learning algorithm. Machine learning may refer to algorithms and statistical models that computer systems may use to perform a specific task without using explicit instructions, instead relying on models and inference. For example, in machine-learning, instead of a rule-based transformation of data, a transformation of data may be used, that is inferred from an analysis of historical and/or training data. For example, the content of images may be analyzed using a machine-learning model or using a machine-learning algorithm. In order for the machine-learning model to analyze the content of an image, the machine-learning model may be trained using training images as input and training content information as output. By training the machine-learning model with a large number of training images and/or training sequences (e.g. words or sentences) and associated training content information (e.g. labels or annotations), the machine-learning model "learns" to recognize the content of the images, so the content of images that are not included in the training data can be recognized using the machine-learning model. The same principle may be used for other kinds of sensor data as well: By training a machine-learning model using training sensor data and a desired output, the machine-learning model "learns" a transformation between the sensor data and the output, which can be used to provide an output based on non-training sensor data provided to the machine-learning model. The provided data (e.g. sensor data, meta data and/or image data) may be preprocessed to obtain a feature vector, which is used as input to the machine-learning model.

Machine-learning models may be trained using training input data. The examples specified above use a training method called "supervised learning". In supervised learning, the machine-learning model is trained using a plurality of training samples, wherein each sample may comprise a plurality of input data values, and a plurality of desired output values, i.e. each training sample is associated with a desired output value. By specifying both training samples and desired output values, the machine-learning model "learns" which output value to provide based on an input sample that is similar to the samples provided during the training. Apart from supervised learning, semi-supervised learning may be used. In semi-supervised learning, some of the training samples lack a corresponding desired output value. Supervised learning may be based on a supervised learning algorithm (e.g. a classification algorithm, a regression algorithm or a similarity learning algorithm. Classification algorithms may be used when the outputs are restricted to a limited set of values (categorical variables), i.e. the input is classified to one of the limited set of values. Regression algorithms may be used when the outputs may have any numerical value (within a range). Similarity learning algorithms may be similar to both classification and regression algorithms but are based on learning from examples using a similarity function that measures how similar or related two objects are. Apart from supervised or semi-supervised learning, unsupervised learning may be used to train the machine-learning model. In unsupervised learning, (only) input data might be supplied and an unsupervised learning algorithm may be used to find structure in the input data (e.g. by grouping or clustering the input data, finding commonalities in the data). Clustering is the assignment of input data comprising a plurality of input values into subsets (clusters) so that input values within the same cluster are similar according to one or more (pre-defined) similarity criteria, while being dissimilar to input values that are included in other clusters.

Reinforcement learning is a third group of machine-learning algorithms. In other words, reinforcement learning may be used to train the machine-learning model. In reinforcement learning, one or more software actors (called "software agents") are trained to take actions in an environment. Based on the taken actions, a reward is calculated. Reinforcement learning is based on training the one or more software agents to choose the actions such, that the cumulative reward is increased, leading to software agents that become better at the task they are given (as evidenced by increasing rewards).

Furthermore, some techniques may be applied to some of the machine-learning algorithms. For example, feature learning may be used. In other words, the machine-learning model may at least partially be trained using feature learning, and/or the machine-learning algorithm may comprise a feature learning component. Feature learning algorithms, which may be called representation learning algorithms, may preserve the information in their input but also transform it in a way that makes it useful, often as a pre-processing step before performing classification or predictions. Feature learning may be based on principal components analysis or cluster analysis, for example.

In some examples, anomaly detection (i.e. outlier detection) may be used, which is aimed at providing an identification of input values that raise suspicions by differing significantly from the majority of input or training data. In other words, the machine-learning model may at least partially be trained using anomaly detection, and/or the machine-learning algorithm may comprise an anomaly detection component.

In some examples, the machine-learning algorithm may use a decision tree as a predictive model. In other words, the machine-learning model may be based on a decision tree. In a decision tree, observations about an item (e.g. a set of input values) may be represented by the branches of the decision tree, and an output value corresponding to the item may be represented by the leaves of the decision tree. Decision trees may support both discrete values and continuous values as output values. If discrete values are used, the decision tree may be denoted a classification tree, if continuous values are used, the decision tree may be denoted a regression tree.

Association rules are a further technique that may be used in machine-learning algorithms. In other words, the machine-learning model may be based on one or more association rules. Association rules are created by identifying relationships between variables in large amounts of data. The machine-learning algorithm may identify and/or utilize one or more relational rules that represent the knowledge that is derived from the data. The rules may e.g. be used to store, manipulate or apply the knowledge.

Machine-learning algorithms are usually based on a machine-learning model. In other words, the term "machine-learning algorithm" may denote a set of instructions that may be used to create, train or use a machine-learning model. The term "machine-learning model" may denote a data structure and/or set of rules that represents the learned knowledge (e.g. based on the training performed by the machine-learning algorithm). In embodiments, the usage of a machine-learning algorithm may imply the usage of an underlying machine- learning model (or of a plurality of underlying machine-learning models). The usage of a machine-learning model may imply that the machine-learning model and/or the data structure/set of rules that is the machine-learning model is trained by a machine-learning algorithm.

For example, the machine-learning model may be an artificial neural network (ANN). ANNs are systems that are inspired by biological neural networks, such as can be found in a retina or a brain. ANNs comprise a plurality of interconnected nodes and a plurality of connections, so-called edges, between the nodes. There are usually three types of nodes, input nodes that receiving input values, hidden nodes that are (only) connected to other nodes, and output nodes that provide output values. Each node may represent an artificial neuron. Each edge may transmit information, from one node to another. The output of a node may be defined as a (non-linear) function of its inputs (e.g. of the sum of its inputs). The inputs of a node may be used in the function based on a "weight" of the edge or of the node that provides the input. The weight of nodes and/or of edges may be adjusted in the learning process. In other words, the training of an artificial neural network may comprise adjusting the weights of the nodes and/or edges of the artificial neural network, i.e. to achieve a desired output for a given input.

Alternatively, the machine-learning model may be a support vector machine, a random forest model or a gradient boosting model. Support vector machines (i.e. support vector networks) are supervised learning models with associated learning algorithms that may be used to analyze data (e.g. in classification or regression analysis). Support vector machines may be trained by providing an input with a plurality of training input values that belong to one of two categories. The support vector machine may be trained to assign a new input value to one of the two categories. Alternatively, the machine-learning model may be a Bayesian network, which is a probabilistic directed acyclic graphical model. A Bayesian network may represent a set of random variables and their conditional dependencies using a directed acyclic graph. Alternatively, the machine-learning model may be based on a genetic algorithm, which is a search algorithm and heuristic technique that mimics the process of natural selection.

As used herein the term "and/or" includes any and all combinations of one or more of the associated listed items and may be abbreviated as "f".

Although some aspects have been described in the context of an apparatus, it is clear that these aspects also represent a description of the corresponding method, where a block or device corresponds to a method step or a feature of a method step. Analogously, aspects described in the context of a method step also represent a description of a corresponding block or item or feature of a corresponding apparatus.

Some embodiments relate to a microscope comprising a system as described in connection with one or more of the Figs. 1 to 5. Alternatively, a microscope may be part of or connected to a system as described in connection with one or more of the Figures 1 to 5. Figure 6 shows a schematic illustration of a system 600 configured to perform a method described herein. The system 600 comprises a microscope 610 and a computer system 620. The microscope 610 is configured to take images and is connected to the computer system 620. The computer system 620 is configured to execute at least a part of a method described herein. The computer system 620 may be configured to execute a machine learning algorithm. The computer system 620 and microscope 610 may be separate entities but can also be integrated together in one common housing. The computer system 620 may be part of a central processing system of the microscope 610 and/or the computer system 620 may be part of a subcomponent of the microscope 610, such as a sensor, an actor, a camera or an illumination unit, etc. of the microscope 610.

The computer system 620 may be a local computer device (e.g. personal computer, laptop, tablet computer or mobile phone) with one or more processors and one or more storage devices or may be a distributed computer system (e.g. a cloud computing system with one or more processors and one or more storage devices distributed at various locations, for example, at a local client and/or one or more remote server farms and/or data centers). The computer system 620 may comprise any circuit or combination of circuits. In one embodiment, the computer system 620 may include one or more processors which can be of any type. As used herein, processor may mean any type of computational circuit, such as but not limited to a microprocessor, a microcontroller, a complex instruction set computing (CISC) microprocessor, a reduced instruction set computing (RISC) microprocessor, a very long instruction word (VLIW) microprocessor, a graphics processor, a digital signal processor (DSP), multiple core processor, a field programmable gate array (FPGA), for example, of a microscope or a microscope component (e.g. camera) or any other type of processor or processing circuit. Other types of circuits that may be included in the computer system 620 may be a custom circuit, an application-specific integrated circuit (ASIC), or the like, such as, for example, one or more circuits (such as a communication circuit) for use in wireless devices like mobile telephones, tablet computers, laptop computers, two-way radios, and similar electronic systems. The computer system 620 may include one or more storage devices, which may include one or more memory elements suitable to the particular application, such as a main memory in the form of random access memory (RAM), one or more hard drives, and/or one or more drives that handle removable media such as compact disks (CD), flash memory cards, digital video disk (DVD), and the like. The computer system 620 may also include a display device, one or more speakers, and a keyboard and/or controller, which can include a mouse, trackball, touch screen, voice-recognition device, or any other device that permits a system user to input information into and receive information from the computer system 620.

Some or all of the method steps may be executed by (or using) a hardware apparatus, like for example, a processor, a microprocessor, a programmable computer or an electronic circuit. In some embodiments, some one or more of the most important method steps may be executed by such an apparatus.

Depending on certain implementation requirements, embodiments of the invention can be implemented in hardware or in software. The implementation can be performed using a non-transitory storage medium such as a digital storage medium, for example a floppy disc, a DVD, a Blu-Ray, a CD, a ROM, a PROM, and EPROM, an EEPROM or a FLASH memory, having electronically readable control signals stored thereon, which cooperate (or are capable of cooperating) with a programmable computer system such that the respective method is performed. Therefore, the digital storage medium may be computer readable.

Some embodiments according to the invention comprise a data carrier having electronically readable control signals, which are capable of cooperating with a programmable computer system, such that one of the methods described herein is performed.

Generally, embodiments of the present invention can be implemented as a computer program product with a program code, the program code being operative for performing one of the methods when the computer program product runs on a computer. The program code may, for example, be stored on a machine readable carrier.

Other embodiments comprise the computer program for performing one of the methods described herein, stored on a machine readable carrier.

In other words, an embodiment of the present invention is, therefore, a computer program having a program code for performing one of the methods described herein, when the computer program runs on a computer.

A further embodiment of the present invention is, therefore, a storage medium (or a data carrier, or a computer-readable medium) comprising, stored thereon, the computer program for performing one of the methods described herein when it is performed by a processor. The data carrier, the digital storage medium or the recorded medium are typically tangible and/or non-transitionary. A further embodiment of the present invention is an apparatus as described herein comprising a processor and the storage medium.

A further embodiment of the invention is, therefore, a data stream or a sequence of signals representing the computer program for performing one of the methods described herein. The data stream or the sequence of signals may, for example, be configured to be transferred via a data communication connection, for example, via the internet.

A further embodiment comprises a processing means, for example, a computer or a programmable logic device, configured to, or adapted to, perform one of the methods described herein.

A further embodiment comprises a computer having installed thereon the computer program for performing one of the methods described herein.

A further embodiment according to the invention comprises an apparatus or a system configured to transfer (for example, electronically or optically) a computer program for performing one of the methods described herein to a receiver. The receiver may, for example, be a computer, a mobile device, a memory device or the like. The apparatus or system may, for example, comprise a file server for transferring the computer program to the receiver.

In some embodiments, a programmable logic device (for example, a field programmable gate array) may be used to perform some or all of the functionalities of the methods described herein. In some embodiments, a field programmable gate array may cooperate with a microprocessor in order to perform one of the methods described herein. Generally, the methods are preferably performed by any hardware apparatus.

### Reference Numerals

- 100: image processing system
- 110: reference image
- 120: target image
- 150: microscope
- 105: deep neural network
- 210: image analysis system
- 250: output image
- 252: predictions
- 310: training image pairs
- 310-1: training reference image
- 310-2: training target image
- 320: predicted target image
- 410-440: method steps for controlling a microscope
- 510-530: method steps for training a deep neural network
- 600: system
- 610: microscope
- 620: computer system
- 710: exemplary reference images
- 720: exemplary training target images
- 730: generated target images

## Claims

1. An image processing system (100) comprising one or more processors and one or more storage devices, wherein the image processing system (100) is configured to:
receive at least one reference image (110), wherein each reference image (110) is a microscopy image capturing cells of a biological sample, wherein the at least one reference image (110) includes at least one reference labeling directed to a reference cellular compartment of the captured cells;
employ a trained deep neural network (105) for processing the at least one reference image (110) to generate a target image (120), wherein the target image (120) includes a target labeling directed to a target cellular compartment of the captured cells, wherein the at least one reference labeling comprises a fluorescence labeling, wherein the reference cellular compartment is a distributed structure within cells, and wherein the target cellular compartment is the cell nucleus.

2. The image processing system (100) of claim 1, wherein the image processing system (100) is further configured to process the target image (120) to obtain centroids and shapes of cell nuclei of the captured cells.

3. The image processing system (100) of claim 1 or claim 2, wherein the at least one reference labeling is based on cytoskeleton markers or cytosolic markers.

4. The image processing system (100) of one of claims 1 to 3, wherein the at least one reference labeling comprises a first fluorescence labeling and a second fluorescence labeling different from the first fluorescence labeling.

5. The image processing system (100) of one of claims 1 to 4, wherein the at least one reference image (110) comprises a plurality of reference images, wherein the plurality of reference images represents a series of optical slices parallel to an optical path of a microscope (150), and wherein the processing the at least one reference image (110) comprises generating a plurality of target images and generating a three-dimensional reconstruction of the target cellular compartment from the plurality of target images.

6. The image processing system (100) of one of claims 1 to 5, further comprising an image analysis system (210) configured to process the target image (120) based on the target labeling, wherein the processing comprises at least one of:
performing a semantic image segmentation to obtain class labels for each pixel in the target image (120),
applying a cell segmentation algorithm on the target image (120),
applying distance transforms and thresholding on the target image (120),
obtaining a count of the captured cells, or
identifying a cell type of the captured cells.

7. The image processing system (100) of one of claims 1 to 6, wherein the image processing system (100) is further configured to process the target image (120) to provide commands to a microscope (150) to least one of:
adapt a control flow of the microscope (150),
change imaging modalities used by the microscope (150), or
change a center field of view of the microscope (150) based on the identified cells.

8. A microscope (150) including the image processing system (100) of one of claims 1 to 7, wherein the microscope (150) is configured to provide the at least one reference image (110) to the image processing system (100) and to display the target image (120).

9. A computer-implemented method (400) for automated image management for a microscope (150), the method (400) comprising:
using (410) the microscope (150) to obtain at least one reference image (110);
sending (420) the at least one reference image (110) to a server comprising the image processing system (100) according to one of claims 1 to 7;
receiving (430) the target image (120) sent from the server at the microscope (150); and
controlling (440) settings of the microscope (150) based on processing the target image (120).

10. A computer-implemented method (500) of training a deep neural network, the method comprising:
receiving (520) training tuples (310) each comprising at least one reference image (310-1) and a target image (310-2), wherein each reference image (310-1) is a microscopy image capturing cells of a biological sample, wherein the at least one reference image (310-1) contains at least one reference labeling directed to a reference cellular compartment of the captured cells and the target image (310-2) contains a target labeling directed to a target cellular compartment of the captured cells, wherein the at least one reference labeling comprises a fluorescence labeling, wherein the reference cellular compartment is a distributed structure within cells, and wherein the target cellular compartment is the cell nucleus; and
training (530) the deep neural network (100) on the training tuples (310) to predict the target image (310-2) from the at least one reference image (310-1).

11. The method of claim 10, wherein the deep neural network (105) is based on a fully convolutional image-to-image neural network, or is based on a visual transformer, or is based on a diffusion model, or is based on an adversarial network.

12. The method of claim 10 or claim 11, wherein the training tuples further comprise bright field microscopy images and phase contrast microscopy images.

13. The method of one of claims 10 to 12, wherein the method further comprises pre-training (510) the deep neural network (105) on pre-training tuples for predicting another target labeling from another reference labeling, the another target labeling and the another reference labeling being different from the target labeling and the reference labeling.

14. The method of claim 13, wherein the deep neural network (105) is based on a variational autoencoder, wherein the method comprises pre-training (510) the deep neural network (105) on the pre-training tuples in a semi-supervised manner.

15. A trained machine learning algorithm (105) trained by:
receiving training tuples (310) each comprising at least one reference image (210-1) and a target image (310-2), wherein each reference image (310-1) is a microscopy image capturing cells of a biological sample, wherein the at least one reference image (310-1) contains at least one reference labeling directed to a reference cellular compartment of the captured cells and the target image (310-2) contains a target labeling directed to a target cellular compartment of the captured cells, wherein the at least one reference labeling comprises a fluorescence labeling, wherein the reference cellular compartment is a distributed structure within cells, and wherein the target cellular compartment is the cell nucleus; and
adjusting the machine learning algorithm based on the training tuples (310) to obtain the trained machine learning algorithm (105).
